# EUROPEAN PATENT APPLICATION

(11) **EP 0 864 570 A1**
(43) Date of publication of application: **16.09.1998**
(21) Application number: 98104063.7
(22) Date of filing: 06.03.1998
(51) Int. Cl.: C07D 405/04

(54) **Epoxy-azetidinones, preparation and use thereof**

(30) Priority: 13.03.1997 HR 970146
(71) Applicant: PLIVA farmaceutska, kemijska, prehrambena i kozmeticka industrija, dionicko drustvo, 10000 Zagreb (HR)
(72) Inventor: Tomic, Mirjana M.Sc., 10000 Zagreb (HR); Kovacevic, Mice D.Sc., 10000 Zagreb (HR)
(74) Representative: von Füner, Alexander, Dr.

(57) **Abstract**

The present invention relates to new epoxy-azetidinones of the general formula I, which are prepared by epoxidizing azetidinones of the general formula II.

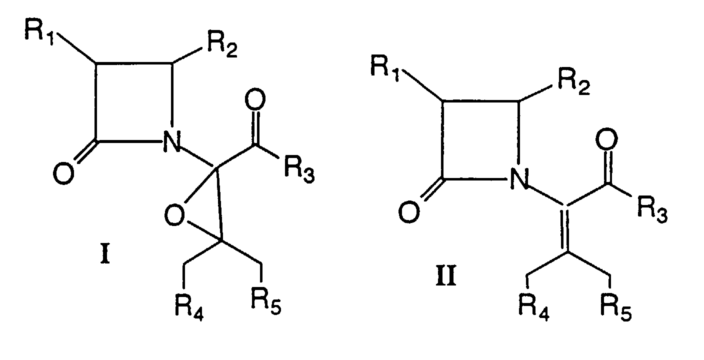

These compounds are useful intermediates for the prepararion of new potential inhibitors of β-lactamases.

## Description

The present invention relates to epoxy-azetidinones, which are new intermediates for the synthesis of 1-oxapenams and other potential inhibitors of β-lactamases.

According to our prior art information it is known that the butenic acid radical at 4-oxoazetidine nitrogen is only converted to epoxide in the case of 2-halo and 2-propenyloxy derivatives. Due to the electrophilic character of alkenes having the double bond in conjugation with electron-acceptor group such as keto groups and carboxy groups there exist so few epoxy-azetidinones, and hence the reaction of alkenes with peroxides and peracids is very difficult to achieve (The Chem. Het. Comp.; Small Ring Het., Part 3, Oxiranes 1985, p. 25).

2-[2-Oxiranylmethoxy-4-oxo-3{[(phenylmethoxy)carbonyl]amino}-1-azetidinyl]-3,3-dimethyloxiranecarboxylic acid diphenylmethyl ester was prepared by researchers of Sionogi and Co.Ltd.Japan as an intermediate in the process for the preparation of 7β-amino-1-oxadethiacephalosporin [DE 2735408 (1978)]. In another known case 2-[2-chloro-4-oxo-3-{[(2,2,2-trichloroetoxy)carbonyl]amino}-1-azetidinyl]-3,3-dimethyloxiranecarboxylic acid methylester was prepared by researchers of Queen's Univ. King. Canada [GB 1,510,795] and also used in the preparation of 1-oxacephem [DE 2531843 (1976)].

In our investigations we have found that also some derivatives of 4-oxoazetidine 2-sulfonic acids having a substituent with double bond conjugated with the carbonyl group of carboxylic acid or of a derivative thereof at azetidine nitrogen, can be transformed to appropriate epoxy-azetidinones. According to our knowledge of the prior art the epoxides of 4-oxoazetidine-2-sulfonic acids and of derivatives thereof are not known.

The object of the present invention are epoxy-azetidinones of the general formula I 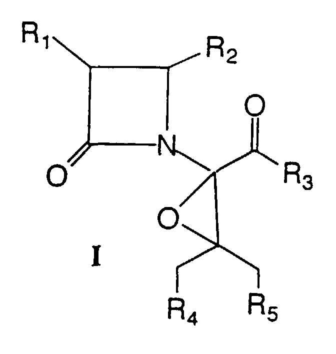 wherein the substituents have the following meaning.
- R¹: is isoxazolylcarbonylamino, phthalimido, phenylacetylamino, phenoxyacetylamino, α-phenylglycylamino, benzyloxycarbonylamino, silylamimo, α-hydroxyethyl and methoxy,
- R²: is alkylaminosulfonyl having 1 to 4 C atoms, benzylaminosulfonyl, isoxazolylaminosulfonyl, chlorosulfonyl, alkyloxysulfonyl having 1 to 4 C atoms, arylsulfonyl, alkyloxy having 1 to 4 C atoms and acyloxy,
- R³: is benzyloxy, alkyloxy having 1 to 4 C atoms, silyloxy, hydroxy, benzylamino, alkylamino having 1 to 4 C atoms, silylamino, amino, halogen and alkyl having 1 to 4 C atoms,
- R⁴ = R⁵: is hydrogen, halogen, alkyloxy having 1 to 4 C atoms, silyloxy, hydroxy and amino.

Another object of the present invention is a process for the preparation of epoxyazetidinones of the general formula I, wherein the said compounds are prepared by epoxidizing azetidinones of the general formula II 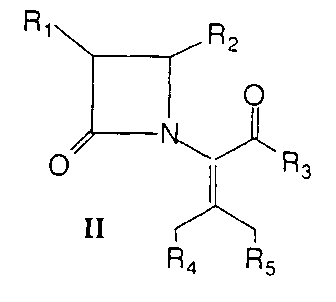 wherein the substituents have the following meaning:
- R¹: is isoxazolylcarbonylamino, phthalimido, phenylacetylamino, phenoxyacetylamino, α-phenylglycylamino, benzyloxycarbonylamino, silylamino, α-hydroxyethyl and methoxy,
- R²: is alkylaminosulfinyl having 1 to 4 C atoms, alkylaminosulfonyl having 1 to 4 C atoms, benzylaminosulfinyl, benzylaminosulfonyl, isoxazolylaminosulfinyl, isoxazolylaminosulfonyl, chlorosulfinyl, chlorosulfonyl, alkyloxysulfinyl having 1 to 4 C atoms, alkyloxysulfonyl having 1 to 4 C atoms, arylsulfonyl, alkyloxy having 1 to 4 C atoms and acyloxy,
- R³: is benzyloxy, alkyloxy having 1 to 4 C atoms, silyloxy, hydroxy, benzylamino, alkylamino having 1 to 4 C atoms, silylamino, amino, halogen and alkyl having 1 to 4 C atoms,
- R⁴ = R⁵: is hydrogen, halogen, alkyloxy having 1 to 4 C atoms, silyloxy, hydroxy and amino.

Compounds of the general formula II can be prepared by means of numerous known processes described and cited in books containing reviews of β-lactam chemistry [The Org. Chem. of β-lactams, 1993; Chem. and Biol. of β-Lactam Antib., 1982; Ceph. and Penic.: Chem. and Biol., 1972]. Further, compounds characterized by the general formula II, wherein R² means alkylaminosulfinyl having 1 to 4 C atoms, alkylaminosulfonyl having 1 to 4 C atoms, benzylaminosulfinyl, benzylaminosulfonyl, isoxazolylaminosulfinyl, isoxazolylaminosulfonyl, chlorosulfinyl, chlorosulfonyl, alkyloxysulfinyl having 1 to 4 C atoms, alkyloxysulfonyl having 1 to 4 C atoms, can be prepared according to the processes described by Takeda Chem. Ind. Ltd. in EP 95764 A3 (1983) and by Pliva, d.d. in US patents 5,250,525 (1993) and 5,466,686 (1995).

Starting from semisynthetic penicillin (cloxacillin) the present invention also describes some new compounds of the general formula II from the class of 4-oxoazetidine-2-sulfinic acids and 4-oxoazetidine-2-sulfonic acids or chlorides and amides thereof, which in general are the object of our previous invention [US patent 5,466,686 (1995), Pliva d.d.] and have now been found transformable to epoxy-azetidinones of the general formula I.

The epoxidizing of thus prepared 4-oxoazetidine-2-sulfinic or 4-oxoazetidine-2-sulfonic acid derivatives is performed with hydrogen peroxide in the presence of formic acid in an organic solvent, mostly ethyl acetate or chlorinated solvents, preferably methylene chloride when it is proceeded with chloroperbenzoic acid. The reaction time depends upon the type of substrate II and a short-term vigorous stirring at room temperature or at a temperature lower than -10°C is mostly required to avoid the degradation of azetidinone ring. It is possible to perform the epoxidizing also by the use of other known reagents described and cited in The Chem. of Het. Comp., Small Ring Het. - Part 3, Oxiranes, 1985, pp. 29-40.

A further object of the present invention is the use of the compounds of the general formula I as intermediates in processes for the preparation of 1-oxapenam compounds and of other β-lactam compounds possessing potential inhibitory activity against β-lactamases. Thereby also 1-oxapenam derivatives of clavulanate type are meant which, in view of the results obtained with clavulanic acid are known as potential inhibitors of β-lactamases.

The present invention is represented by the following Examples and on the basis thereof it can be generally used also for other azetidinone analogs of the general formula II, stated in the description of the invention as well as broader.

### Example 1

### (5R,6R) Benzyl 6-[3'-(o-chlorophenyl)-5'-methyl-4'-isoxazolyl]-carbonylamino-penicillanate

Cloxacillin sodium (10 g, 22 mmol) was dissolved in dimethyl formamide (600 ml) and under stirring benzyl bromide (2.9 ml, 24 mmol) was added to the solution. After stirring at room temperature for 5 hours, the reaction mixture was added dropwise onto an ice-water mixture (600 ml). The resulting suspension was stirred for further 2 hours, the solution was decanted and the solid was dissolved in dichloromethane (200 ml). The solution was dried over Na₂SO₄, filtered and evaporated under reduced pressure. There remained 7.18 g (67.6%) of an oily product. By chromatography on a silica gel column in benzene-ethylacetate solvent system a product having R_{f} = 0.54 (benzene:ethylacetate = 5:1) was separated.
IR (film): 3400m, 1785vs, 1740s, 1670vs, 1600m, 1500m, 1440-1450bm, 1295s, 1200m, 1185m, 750m and 700m cm⁻¹.
300 MHz ¹H NMR(CDCl₃) δ:1.32 (6H, s, CMe₂), 2.78(3H, s, CH₃-isoxazole), 4.33 (1H, s, C₃H), 5.15 (2H, s, CH₂-C₆H₅), 5.44 (1H, d, J=4.32 Hz, C₅H), 5.76 (1H, dd, J=4.3 and 9.3 Hz, C₆H), 5.97 (1H, d, J=9.3 Hz, CONH), 7.57-7.26 (9H, m, C₆H₄ and C₆H₅) ppm.

### Example 2

### (5R,6R) Benzyl 6-[3'-(o-chlorophenyl)-5'-methyl-4'-isoxazolyl]-carbonylamino-penicillanate sulfoxide

(5R,6R) Benzyl 6-[3'-(o-chlorophenyl)-5'-methyl-4'-isoxazolyl]-carbonylamino-penicillanate (7.2 g, 14 mmol) was dissolved in glacial acetic acid (30 ml) and under stirring 30% hydrogen peroxide (2.5 ml) was added thereto. The reaction mixture was stirred for 10 hours at room temperature and then it was added dropwise onto an ice-water mixture (60ml). The resulting suspension was stirred for about 5 hours. The obtained precipitate was filtered, washed with water and dried. The crude product (4.8 g, 66.5%) was crystallized from ethyl acetate; m.p.: 153-155°C; R_{f} 0.36 (benzene:ethyl acetate = 5:1).

| Analysis for C₂₆H₂₄N₃ClO₆S (542.00) | | | | | |
|---|---|---|---|---|---|
| calc.: | C 57.61; | H 4.46; | N 7.75; | Cl 6.54; | S 5.92 % |
| found: | C 57.54; | H 4.51; | N 7.83; | Cl 6.90; | S 5.79 %. |

IR(CH₂Cl₂): 3360s, 1790vs, 1755vs, 1675vs, 1605s, 1500vs, 1455m, 1340m, 1295m, 1270m, 1205vs, 1035m, 765m, 755s, 735s, 700m cm⁻¹.
300 MHz ¹H NMR(CDCl₃) δ: 1.02 and 1.55 (6H, 2s, CMe₂), 2.73 (3H, s, CH₃-isoxazole), 4.53 (1H, s, C₃H), 4.96 (1H, d, J=4.6 Hz, C₅H), 5.14 and 5.26 (2H, 2d, J=11.7 Hz, CH₂-C₆H₅), 6.12 (1H, dd, J=4.6 and 10.3 Hz, C₆H), 6.90 (1H, d, J=10.3 Hz, CONH), 7.27-7.53 (9H, m, C₆H₄ and C₆H₅) ppm.

### Example 3

### (2R,3R) 1-(1'-Benzyloxycarbonyl-2'-methyl-propen-1'-yl)-2-isopropylamino-sulfinyl-3-[3'-(o-chlorophenyl)-5'-methyl-isoxazole-4'-yl]-carbonylamino-4-oxoazetidine

A suspension of CaO (1.22 g, 22 mmol) in toluene (200 ml) was prepared and then by means of Dean-Stark column about 20 ml of the solvent were distilled off. Into a cooled (40°C) suspension (5R,6R) benzyl 6-[3'-(o-chlorophenyl)-5'-methyl-4'-isoxazolyl]-carbonylamino-penicillanate sulfoxide (2.0 g, 3.7 mmol) and N-chlorosuccinimide (0.55 g, 4.15 mmol) were added under stirring and the reaction mixture was stirred under reflux for 90 minutes, cooled to about 15°C and then isopropylamine (0.9 ml. 11 mmol) was added. The stirring was continued at room temperature for further three hours, the mixture was filtered, the filtrate was washed with water (3x30 ml), dried over Na₂SO₄ and evaporated under reduced pressure. The residue was dissolved in dichloromethane (30ml) and evaporated again up to an almost white foamy solid. The crude product (2.06 g, 93%) was purified by chromatography on a silica gel column by means of the solvent mixture dictuoromethane-ethylacetate. Two epimere sulfinamides were separated due to the different configuration on sulphur.

R_{f} = 0.32 (dichloromethane:ethylacetate = 4:1); m.p.: 53°C.
IR (KBr): 4310-3290bm, 1780vs, 1725m, 1675s, 1605m, 1515-1495bm, 1450-1430bm, 1385m, 1365m, 1335m, 1290m, 1210s, 1110m, 1060m, 970m, 695m cm⁻¹.
300 MHz ¹H NMR (CDCl₃): 1.05 (6H, ABq, J=6.3 Hz, CHMe₂), 2.09 and 2.22 (6H, 2s, CMe₂), 3.31-3.38 (2H, m, S-NH and CHMe₂), 4.81 (1H, J=5.4 Hz, C₂H), 5.12 and 5.30 (2H, 2d, J=12.3 Hz, CH₂-C₆H₅), 5.71 (1H, dd, J=5.1 and 9.9 Hz, C₂H), 6.32 (1H, d, J=9.9 Hz, CONH), 7.23-7.54 (9H, m, C₆H₅ and C₆H₄) ppm.

R_{f} 0.22 (dichloromethane:ethylacetate = 4:1); m.p. 65°C.
IR (KBr): 3410-3220bw, 1780vs, 1720m, 1672s, 1600m, 1580-1495bm, 1385m, 1365m, 1290m, 1215s, 1055s, 970w, 755m, 705w cm⁻¹.
300 MHz ¹H NMR (CDCl₃): 1.09 (6H, t, J 6.3 Hz, CHMe₂), 1.96 and 2.24 (6H, 2s, CMe₂), 2,80 (3H, s, Me-isoxazole), 3,38 (1H, m, CHMe₂), 3.86 (1H, J=6.3 Hz, S-NH), 4.66 (1H, d, J=5.1 Hz, C₂H), 5.14 and 5.25 (2H, 2d, J=12.2 Hz, CH₂-C₆H₅), 5.73 (1H, 2d, J=4.8 and 9.4 Hz, C₃H), 6.96 (1H, d, J=9.4 Hz, CONH), 7.26-7.55 (9H, m, C₆H₅ and C₆H₄) ppm.

### Example 4

### (2R,3R) 1-(1'-Benzyloxycarbonyl-2'-methyl-propen-1'-yl)-2-isopropylamino-sulfonyl-3-[3'-(o-chlorophenyl)-5'-methyl-isoxazole-4'-yl]-carbonylamino-4-oxoazetidine

A suspension of (2R,3R) 1-(1'-benzyloxycarbonyl-2'-methyl-propen-1'-yl)-2-isopropylamino-sulfinyl-3-[3'-(o-chlorophenyl)-5'-methyl-isoxazole-4'-yl]-carbonylamino-4-oxoazetidine (4.0 g, 6.5 mmol) in dichloromethane (160 ml) was cooled to 5°C and 30% hydrogen peroxide (35.0 ml) and formic acid (13.0 ml) were added thereto under stirring. The reaction solution was stirred at room temperature, dichloromethane (150 ml) was added thereto and the layers were separated. The organic portion was washed with water, dried over Na₂SO₄ and evaporated under reduced pressure. The crude product (4.0 g, 98%) was subjected to chromatography on a silica gel column by means of the solvent mixture dichloromethane-ethylacetate. There were isolated 3.6 g (89%) of a product having R_{f} = 0.80 (dichloromethane:ethylacetate = 4:1) and m.p. 70-75°C.
IR (CH₂Cl₂): 3400m, 1790vs, 1730s, 1685vs, 1605s, 1520vs, 1440-1425bm, 1390 and 1370m, 1335s, 1290m, 1260m, 1215s, 1060m, 1005m, 895m, 760s, 760m cm⁻¹.
300 MHz ¹H NMR (CDCl₃ ): 1.00 (6H, ABq, J=6.6 Hz, CHMe₂), 1.92 and 2.23 (6H, 2s, CMe₂), 2.78 (3H, s, Me-isoxazole), 3.29 (1H, m, CHMe₂), 3.83 (1H, d, J=8.1 Hz, S-NH), 4.96 (1H, d, J=5.1 Hz, C₂H), 5.07 and 5.30 (2H, 2d, J=12.1 Hz, CH₂-C₆H₅), 5.86 (1H, dd, J=5.1 and 10.2 Hz, C₃H), 6.3 (1H, d, J=10.2 Hz, CONH), 7.27-7.55 (9H, m, C₆H₅ and C₆H₄) ppm.

### Example 5

### (1_{1'}R,2R,3R) 1-(1'-Benzyloxycarbonyl-2'-methyl-1',2'-epoxy-propen-1'-yl)-2-isopropyl--amino-sulfonyl-3-[3'-(o-chlorophenyl)-5'-methyl-isoxazole-4'-yl]-carbonylamino-4-oxo-azetidine

From sulfonamide:
a) (1R,2R) 1-(1'-Benzyloxycarbonyl-2'-methyl-propen-1'-yl)-2-isopropylaminosulfonyl-3-[3'-(o-chlorophenyl)-5'-methyl-isoxazole-4'-yl]-carbonylamino-4-oxoazetidine (2.0 g, 3.25 mmol) was dissolved in dichloromethane (100 ml) and under stirring a solution of m-chloroperbenzoic acid (0.62 g, 3.57 mmol) in dichloromethane (30 ml) was added thereto. The reaction mixture was stirred at room temperature for about 4.0 hours and extracted with a saturated solution of sodium hydrogen carbonate (50 ml). The organic layer was dried over Na₂SO₄, filtered and evaporated under the reduced pressure up to an almost white foamy solid, which was then subjected to chromatography on a silica gel column by means of the solvent mixture dichloromethane-ethylacetate. A product having R_{f} = 0.47 (dichloromethane:ethylacetate = 10:1) and m.p. 142-143 °C (isopropanol) was isolated. m/e 631 (M⁺ + 1).

| Analysis for: C₂₉H₃₁N₄ClO₈S (631.11) | | | | | |
|---|---|---|---|---|---|
| calc.: | C 55.19; | H 4.95; | N 8.88; | Cl 5.62; | S 5.08 %; |
| found: | C 55.00; | H 5.22; | N 8.58; | Cl 5.95; | S 4.86 %. |

IR (CH₂Cl₂): 3400m, 3320s, 1800vs, 1755s, 1735m, 1680vs, 1535-1505bs, 1460m, 1420m, 1375m, 1335s, 1270-1240 bm, 1125s, 1060m, 1005m, 760s, 735s, 700m cm⁻¹.
300 MHz ¹H NMR (CDCl₃) δ: 0.98 and 1.17 (6H, 2d, J=6.6Hz, CHMe₂), 1.43 and 1.52 (6H, 2s, CMe₂), 2.76 (3H, s, Me-isoxazole), 3.47-3.54 (1H, m, CHMe₂), 4.54 (1H, d, J=5.4 Hz, C₂H), 4.80 (1H, d, J=7.7 Hz, S-NH), 5.17 and 5.32 (2H, 2d, J=11.8 Hz, CH₂-C₆H₅), 5.91 (1H, dd, J=5.4 and 10.5 Hz, C₃H), 6.29 (1H, d, J=10.2 Hz, CONH), 7.27-7.51 (9H, m, C₆H₅ and C₆H₄) ppm.

b) The process of Example 5a) was repeated at the temperature of -10°C, whereat a reaction of (1R,2R) 1-(1'-benzyloxycarbonyl-2'-methyl-propen-1'-yl)-2-isopropylamino-sulfonyl-3-[3'-(o-chloro-phenyl)-5'-methyl-isoxazole-4'-yl]-carbonylamino-4-oxo-azetidine within a period of about 6.0 hours occured. After the purification of a crude product on a silica gel column by means of the solvent mixture dichloromethane-ethylacetate, a product identical to the compound described in Example 5a) was isolated.
c) A solution of (1R,2R) 1-(1'-Benzyloxycarbonyl-2'-methyl-propen-1'-yl)-2-isopropylamino-sulfonyl-3-[3'-(o-chlorophenyl)-5'-methyl-isoxazole-4'-yl]-carbonylamino-4-oxo-azetidine (200 mg, 0.32 mmol) in dichloromethane (10 ml) was cooled to 5°C and 30% hydrogen peroxide (2.0 ml) and formic acid (0.60 ml) were added thereto. The reaction mixture was stirred for 3.5 hours at the temperature of 5°C. The layers were separated, the organic layer was washed with water (20 ml), dried over Na₂SO₄, filtered and evaporated under reduced pressure. By chromatography on a silica gel column by means of the solvent mixture dichloromethane-ethylacetate a product identical to the one described in Example 5a) was isolated.

From sulfinamide:
d) To a solution of (1R,2R) 1-(1'-Benzyloxycarbonyl-2'-methyl-propen-1'-yl)-2-isopropylamino-sulfinyl-3-[3'-(o-chlorophenyl)-5'-methyl-isoxazole-4'-yl]-carbonylamino-4-oxo-azetidine (200 mg, 0,33 mmol) in dichloromethane (20 ml), a solution of m-chloroperbenzoic acid (120 mg, 0.70 mmol) was added. The reaction mixture was stirred at room temperature for 5.0 hours and then it was treated as in Example 5a). From the crude product by chromatography on a silica gel column by means of the solvent mixture dichloromethane-ethylacetate a product identical to the one described in Example 5a) was isolated.
e) From the crude product obtained as described in Example 4, by chromatography on a silica gel column by means of the solvent mixture dichloromethane-ethylacetate a substance identical to the one described in Example 5a) was obtained.

## Claims

1. Epoxy-azetidinones of the general formula I 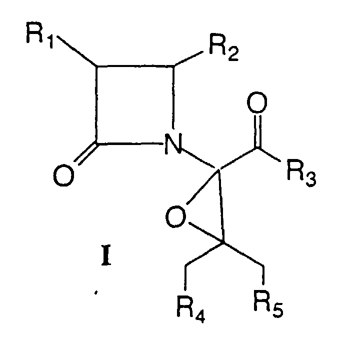 wherein the substituents have the following meanings:
R¹ is 3-o-chlorophenyl-5-methyl-isoxazole-4-yl-carbonylamino, phthalimido, phenylacetylamino, phenoxyacetylamino, α-phenylglycylamino, benzyloxycarbonylamino, silylamino, α-hydroxyethyl and methoxy,
R² is alkylaminosulfonyl having 1 to 4 C atoms, benzylaminosulfonyl, 5-methylisoxazole-3-yl-aminosulfonyl, chlorosulfonyl, alkyloxysulfonyl having 1 to 4 C atoms, arylsulfonyl, alkyloxy having 1 to 4 C atoms and acyloxy,
R³ is benzyloxy, alkyloxy having 1 to 4 C atoms, silyloxy, hydroxy, benzylamino, alkylamino having 1 to 4 C atoms, silylamino, amino, halogen and alkyl having 1 to 4 C atoms,
R⁴ = R⁵ is hydrogen, halogen, alkyloxy having 1 to 4 C atoms, silyloxy, hydroxy and amino.

2. Compound according to claim 1, characterized in that R¹ is 3-o-chlorophenyl-5-methyl-isoxazole-4-yl-carbonylamino, R² is isopropylaminosulfonyl, R³ is benzyloxy and R⁴ = R⁵ is hydrogen.

3. Compound according to claim 1, characterized in that R¹ is 3-o-chlorophenyl-5-methyl-isoxazole-4-yl-carbonylamino, R² is isopropylaminosulfonyl, R³ is hydroxy and R⁴ = R⁵ is hydrogen.

4. Compound according to claim 1, characterized in that R¹ is 3-o-chlorophenyl-5-methyl-isoxazole-4-yl-carbonylamino, R² is isopropylaminosulfonyl, R³ is benzylamino and R⁴ = R⁵ is hydrogen.

5. Compound according to claim 1, characterized in that R¹ is 3-o,o'-dichlorophenyl-5-methyl-isoxazole-4-yl-carbonylamino, R² is isopropylaminosulfonyl, R³ is benzyloxy and R⁴ = R⁵ is hydrogen.

6. Compound according to claim 1, characterized in that R¹ is 3-o-chlorophenyl-5-methyl-isoxazole-4-yl-carbonylamino, R² is 5-methyl-isoxazole-3-yl-aminosulfonyl, R³ is benzyloxy and R⁴ = R⁵ is hydrogen.

7. Compound according to claim 1, characterized in that R¹ is 3-o-chlorophenyl-5-methyl-isoxazole-4-yl-carbonylamino, R² is chlorosulfonyl, R³ is benzyloxy and R⁴ = R⁵ is hydrogen.

8. Compound according to claim 1, characterized in that R¹ is 3-o-chlorophenyl-5-methyl-isoxazole-4-yl-carbonylamino, R² is methoxy, R³ is benzyloxy and R⁴ = R⁵ is hydrogen.

9. Compound according to claim 1, characterized in that R¹ is 3-o-chlorophenyl-5-methyl-isoxazole-4-yl-carbonylamino, R² is acetyl, R³ is benzyloxy and R⁴ = R⁵ is hydrogen.

10. A process for the preparation of epoxy-azetidinones of the general formula I, wherein the radicals have the meanings as defined in claim 1, characterized in that azetidinones of the general formula II 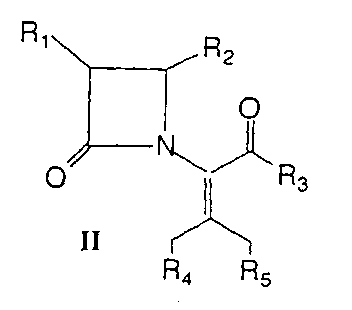 wherein the substituents have the following meanings:
R¹ is 3-o-chlorophenyl-5-methyl-isoxazole-4-yl-carbonylamino, phthalimido, phenylacetylamino, phenoxyacetylamino, α-phenylglycylamino, benzyloxycarbonylamino, silylamino, α-hydroxyethyl and methoxy,
R² is alkylaminosulfinyl having 1 to 4 C atoms, isopropylaminosulfonyl, 5-methyl-isoxazole-3-yl-aminosulfonyl, benzylaminosulfonyl, chlorosulfinyl, chlorosulfonyl, methoxy, acetyl, methylsulfonyl and methylthio, methyloxysulfonyl, arylsulfonyl,
R³ is benzyloxy, alkyloxy having 1 to 4 C atoms, silyloxy, hydroxy, benzylamino, alkylamino having 1 to 4 C atoms, silylammo, amino, halogen and alkyl having 1 to 4 C atoms,
R⁴ = R⁵ is hydrogen, halogen, alkyloxy having 1 to 4 C atoms, silyloxy, hydroxy and amino,
are subjected to the reaction of epoxidizing.

11. Use of epoxy-azetidinones of the general formula I as intermediates for the preparation of new oxapenam analogs, especially of potential inhibitors of β-lactamases.
